# EUROPEAN PATENT SPECIFICATION

(11) **EP 0 644 783 B1**
(45) Date of publication and mention of the grant of the patent: **17.12.1997**
(21) Application number: 93912425.1
(22) Date of filing: 11.06.1993
(51) Int. Cl.: A61B 19/02, A61M 5/32, A61M 5/00, A61B 19/00

(54) **APPARATUS FOR THE DISPOSAL OF SHARPS**
VORRICHTUNG FÜR DIE ENTSORGUNG VON SCHARFEN GEGENSTÄNDEN
APPAREIL D'ELIMINATION D'OBJETS AFFILES

(30) Priority: 11.06.1992 AU PL2891/92
(43) Date of publication of application: 29.03.1995
(73) Proprietor: ZALSTROVS, Ivars, Kewdale, W.A. 6105 (AU)
(72) Inventor: ZALSTROVS, Ivars, Kewdale, W.A. 6105 (AU)
(74) Representative: W.P. Thompson & Co.
(86) International application number: AU9300275
(87) International publication number: WO9325250

(56) References cited:
- EP-A- 0 136 392
- EP-A- 0 262 686
- EP-A- 0 332 584
- EP-A- 0 455 075
- AU-A- 1 742 792
- DE-C- 4 221 153
- GB-A- 2 211 420
- US-A- 4 877 934
- US-A- 5 212 362

## Description

The present invention relates to an apparatus and method for the destruction of sharps such as hypodermic needles or blood lances.

### FIELD OF THE INVENTION

Hypodermic needles and lances are used for the injection and extraction of fluids from organisms. Because of difficulties associated with sterilization for subsequent reuse of needles, it is generally preferred that sharps are only used once before being discarded. Used sharps may contain or have been in close contact with highly infectious bacteria and viruses or other substances considered harmful. Further, the presence of fluids extracted from organisms on sharps can provide material for the growth and multiplication of other harmful agents which could potentially be transmitted to those handling the sharps, particularly if the sharps are re-used or where the sharp accidentally punctures the skin.

It is thus desirable to ensure that sharps cannot be re-used and that an effective means of sterilising sharps is employed.

Devices which destroy hypodermic syringes by shearing the needle have been proposed to prevent the re-use of the needle. Whilst these devices are certainly effective in preventing a second use of the needle they have no sterilizing effect and any infectious or harmful material remains on the sharp. Moreover, those handling the sharp are still exposed to the possibility of accidental injury through the sharp puncturing the skin.

Devices which destroy the sharp by electric current have also been proposed (WO-A-9219291). Sharps generally have a high surface area and a relatively low volume. Resistance to the passage of high current will thus cause heating of the sharp. A sufficiently high current will cause the sharp to fuse thereby destroying the sharp.

Devices which operate on the electrical resistance principle have to overcome a number of difficulties. In particular, problems have been encountered in destroying the needle completely without leaving a remnant stub attached to the needle. Attempts to avoid this problem have generally involved various configurations of electrodes which permit a needle to be inserted therebetween to destroy the needle. During the destruction process the remaining portion of the needle becomes successively shorter and more difficult to destroy.

Electrodes are generally shaped so as to have the smallest possible gap therebetween which allows short portions of needle to be destroyed. However, this approach to the problem of needle destruction is not without difficulties.

Firstly, by having the electrodes spaced very closely together it becomes difficult for an operator to locate the closely spaced gap with the needle to be destroyed. Thus, a portion of the needle will not be destroyed and the risk of injury and/or infection from such a needle may remain.

Secondly, as a needle is being destroyed a residue of fused needle material is produced. Ideally, the residue should pass between the electrodes and usually fall into a collection drawer located below the electrodes. However, where the electrodes approach one another very closely there may be insufficient space for the residue to fall through and the electrode gap can thus become congested with residue. In an attempt to avoid this problem users of devices are commonly encouraged to rotate and jiggle the needle during destruction. However, in practice this is often overlooked and the electrodes become blocked. The result being that the electrodes have to be removed from the device and cleared or replaced.

Thirdly, the geometry of the electrodes usually prohibits the use of the device for any item other than a needle.

Thus, blood lances, for example, cannot usually be destroyed by such devices.

The present invention seeks to substantially ameliorate the above mentioned problems by providing a device which allows the electrodes to be moved relative to each other and in particular, which allows the electrodes to approach each other more closely as the portion of a needle close to the needle hub is destroyed.

### SUMMARY OF THE INVENTION

In accordance with a first aspect of the present invention there is provided an apparatus for the destruction of sharps having shafts of fusible conductive material retained in a retaining body, the apparatus comprising a pair of spaced electrodes arranged to be connected to a power supply, characterised in that the electrodes are disposed such that at least one of the electrodes is pivotally movable relative to the other electrode so as to allow a portion of a shaft of fusible conductive material to be placed between the electrodes to complete an electrical circuit so as to allow the portion to be destroyed and to allow successive portions of the shaft to be placed between the electrodes until all of the shaft is destroyed and the retaining body abuts the electrodes so as to cause at least one electrode to pivot towards the other electrode.

### BRIEF DESCRIPTION OF THE DRAWINGS

The present invention will now be described, by way of example, with reference to the accompanying drawings, in which:
Figure 1 represents a sectional side view of an apparatus in accordance with a first embodiment of the present invention;
Figures 2a,b and c illustrate in detail the electrodes of the apparatus of Figure 1 during the destruction of a hypodermic needle;
Figure 3 illustrates the electrode component of a second embodiment of the present invention; and
Figure 4 illustrates a schematic sectional side view of a third embodiment of the present invention.

### DESCRIPTION OF THE INVENTION

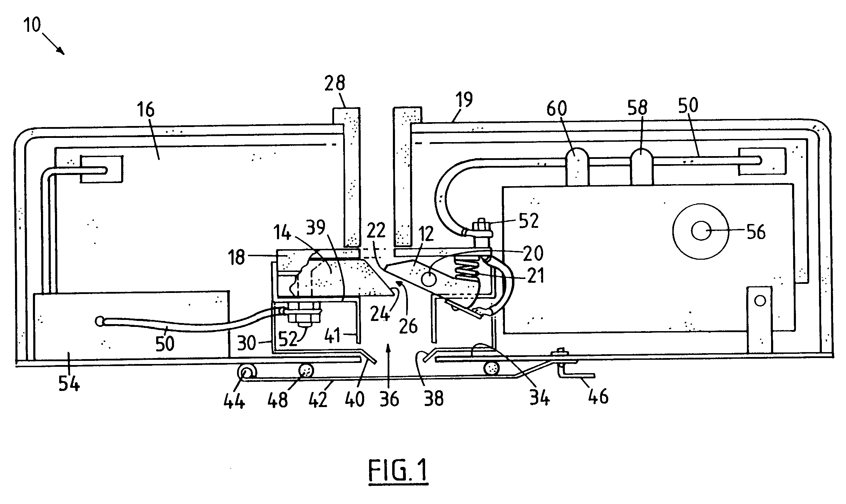

Illustrated in Figure 1 is an apparatus for the destruction of sharps 10 comprising first and second spaced electrodes 12,14, each electrode 12,14 being connected to a power supply 16. The power supply 16 is typically of a high frequency AC power supply. Alternatively, with the aid of suitable circuitry DC power from a battery may be converted to high frequency power.located in an electrode housing 18 only partially shown for the sake of clarity. The electrodes 12,14 and power supply 16 are all contained within a housing 19. The first electrode 12 is pivotally connected to the electrode housing 18 by pivot 20. The first electrode 12 is inclined upwardly relative to the second electrode 14 and may be moved downwardly in an arc centred on the pivot 20 towards the second electrode 14.

Further, a spring 21 abutting an upper surface of the electrode 12 resiliently biases the electrode 12 into position against the electrode housing 18.

The second electrode 14 is connected to the electrode housing 18 by means of a screw.

Each of the first and second electrodes 12,14 has a respective inclined inner face 22,24. The inclined inner faces 22,24 form a channel 26 passing between the electrodes 12,14. The inner faces 22,24 converge at lower ends thereof.

A removable open ended tube 28 is located directly above the electrode housing 18 and extends through the apparatus housing 19. The guide tube 28 is of sufficient diameter to allow a small gauge hypodermic syringe and needle hub to be inserted therethrough. The guide tube 28 is replaceable with alternative guide tubes to suit other needle gauges.

The electrode housing 18 rests on top of a residue container 30. The residue container 30 forms a barrier between any debris resulting from the destruction of a needle and the components of the apparatus 10. Below the residue container 30 a base plate 34 containing an aperture 36 is located directly below the electrodes 12,14. A downwardly inclined lip 38 extends around the aperture 36 and around a concentric aperture 40 in the apparatus housing 19.

In the example described the apparatus is to be used with small gauge needles. Where larger needles are to be destroyed a debris collection drawer may be employed to collect the residue.

Attached to the underside of the electrode housing 18 are a pair of defecting skirts 39. The deflect skirts 39 extend inwardly beneath the electrodes 12, 14 and terminate in downwardly depending flanges 41 located either side of the channel 26.

Below the apertures 36 and 40 and in spaced apart relation thereto is a shelf 42. The shelf 42 is hingedly connected to the apparatus housing 19 by hinge 44 and secured in a generally horizontal position by twist lock 46. The purpose of the shelf 42 is to act as a means for a collection of the debris and prevent any material from falling out should the apparatus be inverted.

A seal between the shelf 42 and housing 19 is achieved by o-ring 48 located around the apertures 36 and 40.

The power supply 16 is in connection to the electrodes 12,14 by means of power leads 50. The power leads 50 are connected to terminal posts 52 located in the electrode housing 18. The pivot point 20 in the electrode 12 may also serve as a terminal post 52. Further, a protective circuit breaker 54 is placed in the circuit between the power supply 16 and the electrodes 12,14. Further, LED indicators 58,60 indicate the operational state of the unit.

The apparatus 10 may additionally contain a timer circuit (not shown) to operate a filtering device. Similarly, an electronic circuit interruption may be included to switch off the apparatus 10 if inappropriate objects such as scissors are inserted between the electrodes 12, 14. The circuit interruption also operates in overcurrent situations, such as when the electrodes touch one another.

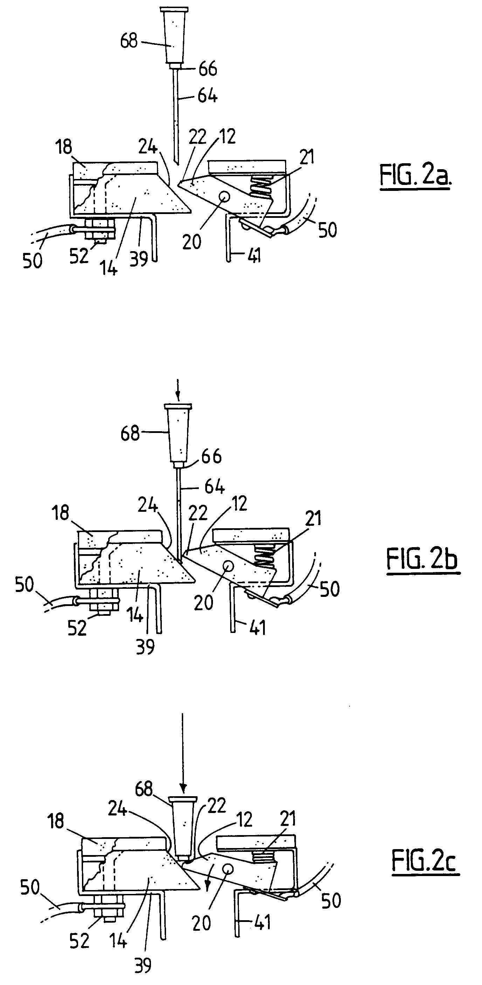

In use, a hypodermic syringe or blood lance is inserted into the guide tube 28 until contact with the electrodes 12,14 is made. The destruction of the needle of hypodermic syringe comprising a metallic needle 64, plastic hub 66 and body 68 is illustrated in detail in Figures 2a, b and c.

For clarity only, details of the electrode housing 18 and electrodes 12,14 are included. The needle 64 is inserted vertically in the guide tube 28 until the needle 64 contacts the electrodes 12,14. A portion 64a of the needle 64 between the electrodes 12,14 completes an electrical circuit and is heated by the current passing through the portion 64a. The portion 64a is heated until it fuses.

The material then falls through the apertures 36,40 and onto the shelf 42.

Successive portions of the needle 64 are destroyed until the hub 66 approaches the electrodes 12,14. The hub 66 may be brought to bear on the first electrode 12 thus causing the electrode 12 to move on an arc around the pivot 20 and against the spring 21. As can be seen in Figure 2c the gap between the electrodes 12,14 is thus narrowed considerably allowing the destruction of the portion of the needle 64 close to the hub 66. When the needle 64 is destroyed the syringe may be lifted relieving the pressure on the electrode 12. The electrode 12 is now urged into the original position by the spring 21. Thus, the channel 26 between the electrodes 12,14 is now sufficiently wide for any residue to fall into the residue container 30.

The apparatus of the present invention as described herein has a number of further advantages that become apparent when the device is used.

When residue material falls through the apertures 36,40 and the flanges 41 and tip 38 act as a baffle and generally restrict any movement of the residue back through the apertures 36,40 and channel 26. Thus, the apparatus 10 may be inverted without shedding any of the residue.

Similarly, where a mains rechargable battery is incorporated the apparatus 10 may be used remote from a power supply.

Both of the above features are of considerable benefit to users who require a portable unit such as diabetics who wish to destroy needles which have been used for insulin injections. However, it is to be understood that the apparatus of the present invention may also be devised in a form which allows connection to a mains power supply.

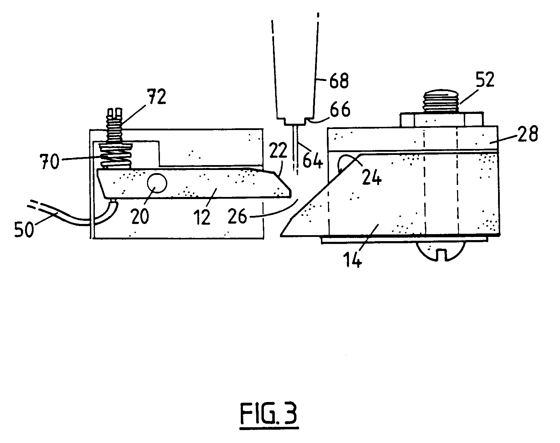

Figures 2 and 3 illustrate, schematically, further embodiments of the present invention. Accordingly, like reference numerals have been used to indicate like parts.

Figure 3 depicts the electrode component only of a second embodiment of the present invention. In this embodiment, the first electrode 12 is resiliently biased into position by a spring 70 located above the electrode and remote from the inner face 22. Further, the spring 70 abuts an adjustable screw 72 such that the pressure exerted on the electrode 12 may be varied and the spring 72 may be used to restrict the travel of the electrode 12. Thus in use, when the needle 64 and needle hub 66 are urged against the electrode 12 the pressure required to cause the electrode 12 to move about pivot 20 may be adjusted by an adjustment of the screw 72. The arrangement of the spring 70 and screw 72 may be particularly useful where a wide range of needle gauges is to be used.

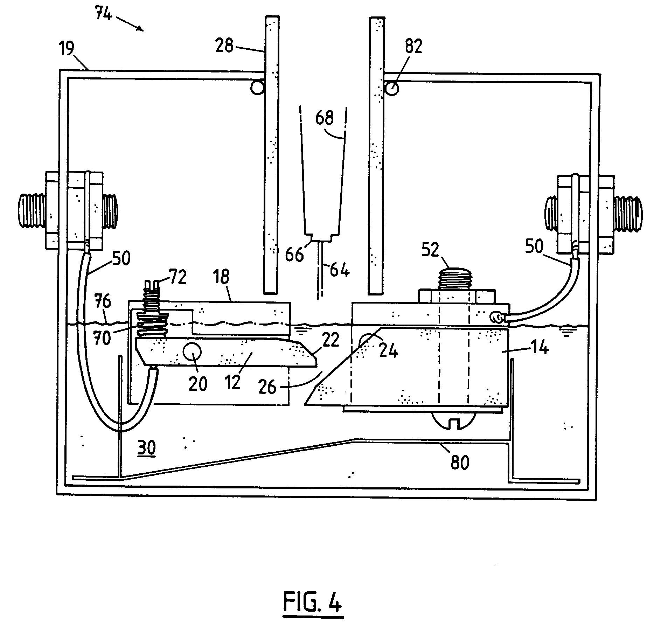

Figure 4 illustrates an apparatus for the destruction of sharps 74 in which the electrodes are immersed in a non conductive fluid 76. The fluid 76 has the effect of reducing the smoke and/or sparks which are generated during the operation of the device and which can be unsettling for operators.

Further, to enable the apparatus 74 to be constructed with only a minimum volume of fluid used the residue container 30 is entirely sealed at the base and has an angled spacer 80 to reduce the volume of fluid required. The spacer 80 is angled downwardly from the channel 26 to encourage the residue to fall away from the electrodes 12,14.

The fluid used is preferably antiseptic and of a high viscosity, high boiling point and low volatility to reduce movement of fluid when the apparatus 74 is being moved or inverted.

The apparatus 74 may also be provided with a needle detection means 82. The needle detection means may operate on either optical magnetic or capacitative means and may be used to ensure that the apparatus is only operational when a needle or other sharp to be destroyed is present.

In other alternative embodiments of the present invention the spring 21 may be replaced by other resilient means such as resiliently biasable strips of brass or phosphor bronze.

Modifications and variations such as would be apparent to a skilled addressee are deemed within the scope of the present invention.

## Claims

1. An apparatus for the destruction of sharps (10) having shafts (64) of fusible conductive material retained in a retaining body (66), the apparatus comprising a pair of spaced electrodes (12, 14) arranged to be connected to a power supply (16), characterised in that the electrodes are disposed such that at least one of the electrodes (12) is pivotally movable relative to the other electrode (14) so as to allow a portion of a shaft (64) of fusible conductive material to be placed between the electrodes to complete an electrical circuit so as to allow the portion to be destroyed and to allow successive portions of the shaft to be placed between the electrodes until all of the shaft is destroyed and the retaining body abuts the electrodes so as to cause at least one electrode to pivot towards the other electrode.

2. An apparatus for the destruction of sharps according to claim 1, characterised in that at least one electrode (12) is resiliently biased into position.

3. An apparatus for the destruction of sharps according to claim 1, characterised in that each electrode has an inner face (22, 24) adjacent the other electrode inclined such that a gap between the electrodes becomes less towards lower edges of the electrodes.

4. An apparatus for the destruction of sharps according to claim 1, characterised in that the or each pivotally movable electrode may be moved around the pivot (20) by pressure brought to bear on the electrode by the retaining body such that the electrodes are urged together and a gap between the electrodes is reduced allowing portions of the shaft close to the retaining body to be destroyed.

5. An apparatus for the destruction of sharps according to claim 4, characterised in that following the destruction of a sharp when the pressure is removed from the or each pivotally moveable electrode the electrode is biassed into position so as to allow debris to fall through a channel (26) between the electrodes.

6. An apparatus for the destruction of sharps according to claim 1, characterised in that the apparatus has a plurality of deflection means (38, 40) located below the electrodes to guide any debris.

7. An apparatus for the destruction of sharps according to claim 1, characterised in that the apparatus further comprises a means (76) for reducing the effect of sparks and smoke generated during the use of the apparatus.

8. An apparatus for the destruction of sharps according to claim 1, characterised in that the relative positions of the electrodes is adjustable.

## Patentansprüche

1. Vorrichtung zum Zerstören von scharfen Gegenständen (10) mit in einem Haltekörper (66) gehaltenen Schäften (64) aus schmelzbarem leitfähigem Material, wobei die Vorrichtung ein Paar beabstandeter Elektroden (12, 14) umfaßt, die zum Verbinden mit einer Stromversorgung (16) angeordnet sind, dadurch gekennzeichnet, daß die Elektroden derart angeordnet sind, daß mindestens eine der Elektroden (12) im Verhältnis zu der anderen Elektrode (14) drehbar beweglich ist, so daß ein Teil eines Schaftes (64) aus schmelzbarem leitfähigem Material zwischen die Elektroden gebracht werden kann, um einen elektrischen Stromkreis zu schließen, so daß der Teil zerstört werden kann und nachfolgende Teile des Schaftes zwischen die Elektroden gebracht werden können, bis der ganze Schaft zerstört worden ist und der Haltekörper an den Elektroden anliegt, so daß bewirkt wird, daß mindestens eine Elektrode auf die andere Elektrode zu gedreht wird.

2. Vorrichtung zum Zerstören von scharfen Gegenständen nach Anspruch 1, dadurch gekennzeichnet, daß mindestens eine Elektrode (12) federnd in ihre Position vorgespannt wird.

3. Vorrichtung zum Zerstören von scharfen Gegenständen nach Anspruch 1, dadurch gekennzeichnet, daß jede Elektrode eine an die andere Elektrode angrenzende Innenseite (22, 24) hat, die derart geneigt ist, daß ein Spalt zwischen den Elektroden zu unteren Rändern der Elektroden hin kleiner wird.

4. Vorrichtung zum Zerstören von scharfen Gegenständen nach Anspruch 1, dadurch gekennzeichnet, daß die oder jede drehbar bewegliche Elektrode durch von dem Haltekörper auf die Elektrode aufgebrachten Druck derart um den Drehpunkt (20) herum bewegt werden kann, daß die Elektroden aufeinander zu bewegt werden und ein Spalt zwischen den Elektroden verkleinert wird, wodurch Teile des Schaftes nahe am Haltekörper zerstört werden können.

5. Vorrichtung zum Zerstören von scharfen Gegenständen nach Anspruch 4, dadurch gekennzeichnet, daß die Elektrode nach dem Zerstören eines scharfen Gegenstands, wenn der Druck von der oder jeder drehbar beweglichen Elektrode genommen wird, in eine Position vorbelastet wird, um Abfall durch eine Rinne (26) zwischen den Elektroden hindurchfallen zu lassen.

6. Vorrichtung zum Zerstören von scharfen Gegenständen nach Anspruch 1, dadurch gekennzeichnet, daß die Vorrichtung eine Mehrzahl von unter den Elektroden angeordneten Ablenkeinrichtungen (38, 40) zum Lenken von Abfall hat.

7. Vorrichtung zum Zerstören von scharfen Gegenständen nach Anspruch 1, dadurch gekennzeichnet, daß die Vorrichtung ferner eine Einrichtung (76) umfaßt zum Vermindern der Auswirkungen von während der Benutzung der Vorrichtung erzeugtem Rauch und Funken.

8. Vorrichtung zum Zerstören von scharfen Gegenständen nach Anspruch 1, dadurch gekennzeichnet, daß die relativen Positionen der Elektroden verstellbar sind.

## Revendications

1. Appareil pour la destruction d'objets affilés (10) qui ont une tige (64) en matériau conducteur fusible retenue dans un corps de retenue (66), l'appareil comprenant une paire d'électrodes espacées (12, 14) agencées de manière à être connectées à une alimentation (16), caractérisé en ce que les électrodes sont disposées de telle sorte qu'au moins une des électrodes (12) est mobile en rotation par rapport à l'autre électrode (14), de manière à permettre de placer une partie d'une tige (64) en matériau conducteur fusible entre les électrodes pour compléter un circuit électrique afin de permettre la destruction de la partie, et de manière à permettre de placer des parties successives de la tige entre les électrodes jusqu'à ce que toute la tige soit détruite et que le corps de retenue vienne en butée contre les électrodes pour provoquer le pivotement d'au moins une électrode vers l'autre électrode.

2. Appareil pour la destruction d'objets affilés selon la revendication 1, caractérisé en ce qu'au moins une électrode (12) est sollicitée de manière élastique en position.

3. Appareil pour la destruction d'objets affilés selon la revendication 1, caractérisé en ce que chaque électrode a une face intérieure (22, 24) adjacente à l'autre électrode inclinée de telle manière qu'un espace entre les électrodes diminue vers les bords inférieurs des électrodes.

4. Appareil pour la destruction d'objets affilés selon la revendication 1, caractérisé en ce que la ou chaque électrode mobile en rotation peut être déplacée autour du pivot (20) par une pression exercée sur l'électrode par le corps de retenue, de manière à pousser les électrodes l'une vers l'autre et à réduire un espace entre les électrodes pour permettre la destruction de parties de la tige proches du corps de retenue.

5. Appareil pour la destruction d'objets affilés selon la revendication 4, caractérisé en ce que, après la destruction d'un objet affilé, lorsque la pression n'est plus exercée sud la ou chaque électrode mobile en rotation, l'électrode est sollicitée en position de manière à permettre à des débris de tomber à travers un canal (26) entre les électrodes.

6. Appareil pour la destruction d'objets affilés selon la revendication 1, caractérisé en ce que l'appareil a une pluralité de moyens de déflexion (38, 40) situés sous les électrodes pour guider des débris.

7. Appareil pour la destruction d'objets affilés selon la revendication 1, caractérisé en ce que l'appareil comprend en outre des moyens (76) pour réduire l'effet des étincelles et de la fumée générées pendant l'utilisation de l'appareil.

8. Appareil pour la destruction d'objets affilés selon la revendication 1, caractérisé en ce que la position relative des électrodes peut être réglée.
